# EUROPÄISCHE PATENTSCHRIFT

(11) **EP 0 761 636 B1**
(45) Veröffentlichungstag und Bekanntmachung des Hinweises auf die Patenterteilung: **03.11.1999**
(21) Anmeldenummer: 96113887.2
(22) Anmeldetag: 30.08.1996
(51) Int. Cl.: C07C 55/14, C07C 51/47

(54) **Verfahren zur Wiedergewinnung von Katalysatoren bei der Adipinsäure-Herstellung**
Recovery of catalysts from adipic acid production
Récupération de catalyseurs à partir de préparation d'acide adipique

(30) Priorität: 12.09.1995 DE 19533688
(43) Veröffentlichungstag der Anmeldung: 12.03.1997
(73) Patentinhaber: BAYER AG, 51368 Leverkusen (DE)
(72) Erfinder: Salzburg, Herbert, Dr., 42799 Leichlingen (DE); Steinhoff, Georg, Dr., 47800 Krefeld (DE); Hoffmann, Heiko, 51427 Bergisch Gladbach (DE); Kaponig, Helmut, 46049 Oberhausen (DE)

(56) Entgegenhaltungen:
- US-A- 2 858 335
- US-A- 3 965 164
- US-A- 5 182 251

## Beschreibung

Die Erfindung betrifft ein Verfahren zur Wiedergewinnung von Katalysatoren bei der Adipinsäure-Herstellung.

Adipinsäure wird großtechnisch z.B. durch katalytische Oxidation von Cyclohexanol hergestellt. Als Katalysatoren können Cu- und Vanadiumsalze eingesetzt werden, als Oxidationsmittel konzentrierte Salpetersäure.

Die Reaktionslösung aus Katalysator und Oxidationsmittel reichert sich mit Eisen-Ionen an (Eisenabtrag aus den Reaktionsgefäßen). Dieser Eisengehalt kann eine e weitere Verwendung der Reaktionslösung stören oder verhindern.

Es ist nun eine Reihe von Verfahren bekannt, bei denen die Metalle der Prozeßlösung mit Ionenaustauschern entzogen werden. Dies geschieht üblicherweise bei der Aufarbeitung der Nebenprodukte aus der Adipinsäuresynthese (Glutarsäure, Bernsteinsäure). In der US-A 3.965.164 ist die Behandlung der Gesamtmischung von Metallen und Dicarbonsäuren mit Prozeßabgas (NOₓ, N₂O) beschrieben, wodurch eine Vanadin-Aufkonzentrierung beim Regenerieren des Ionenaustauschers erreicht wird.

In der EP-A 0 494 416 wird z.B. die Zersetzung der Metallnitrate durch thermische Behandlung beschrieben. Als Folge hiervon ist durch den Ionenaustauscher kein Eisen, wohl aber Vanadin und Kupfer durch Kationenaustausch abgetrennt worden.

Eine weitere Möglichkeit zur Trennung von Kupfer und Vanadin einerseits und Eisen andererseits besteht darin, bei der Regenerierung des Ionenaustauschers mit schwacher Salpetersäure vorzuspülen, um Cu und V bevorzugt zu eluieren, und anschließend das Eisen (sowie Reste von Cu und V) mit stärkerer HNO₃ abzuwaschen (SU 690320).

Bei einer weiteren Methode (US 3 554 692-S) wird Kupfer und Vanadin mit HNO₃ (pH 1,8 bis -0,3) selektiv vom Ionenaustauscher gewaschen, das Eisen dagegen als Phosphat-Komplex eluiert.

Bei der selektiven Regenerierung des Ionenaustauschers durch verdünnte Säuren ist nachteilig, daß naturgemäß große Mengen an Regeneriersäure aufzuwenden sind. Bei der Verwendung von Hilfschemikalien ist nachteilig, daß dem Oxidationsprozeß bei der Katalysator-Wiederverwendung eine "fremde" Substanz zugeführt werden kann, so daß eine Reinigung der Kupfer- und Vanadinverbindungen vor Wiederverwendung erforderlich wird. Darüber hinaus besteht ein allgemeines Problem darin, daß die Eisen-Konzentrationen wesentlich geringer sind als die Kupfer- und Vanadin-Konzentrationen, so daß schon die Wahrscheinlichkeit des bevorzugten Eisenaustauschs stark beeinträchtigt wird.

Die Verfahrensarten haben zusätzlich den Nachteil, daß ein mehr oder weniger großer Eisenschlupf bei der selektiven Regenerierung hingenommen werden muß, wenn der Kupfer- und Vanadinverlust nicht zu groß werden soll.

Es wurde nun ein Verfahren gefunden, bei dem aus einer Reaktionslösung zur Oxidation von Cyclohexanol zu Adipinsäure mit Kupfer- und Vanadiumsalzen als Katalysatoren und konzentrierter Salpetersäure Eisenionen entfernt werden können, welche z.B. durch Abtrag aus den Reaktionsgefäßen stammen.

Gegenstand der Erfindung ist ein Verfahren zur Entfernung von Eisenionen aus einer Reaktionslösung wie sie bei der Oxidation von Cyclohexanol mit Salpetersäure zu Adipinsäure mit konzentrierter Salpetersäure in Gegenwart von Kupfer-und Vanadiumsalzen anfällt, dadurch gekennzeichnet, daß man die Reaktionslösung nach Abtrennen der Adipinsäure über einen sulfonierten Ionenaustauscher leitet, wobei alle Metallionen absorbiert werden, daß man anschließend den beladenen Ionenaustauscher abtrennt, mit Salpetersäure alle absorbierten Metallionen auswäscht und das Eluat auf einen mit Aminophosphonsäuregruppen modifizierten Ionenaustauscher aufgibt, wobei die Eisenionen selektiv abgetrennt werden.

Zur erfindungsgemäßen Entfernung aller Metallionen aus der Reaktionslösung der Adipinsäureherstellung wird diese über einen sulfonierten Polystyrolionenaustauscher, z.B. Lewatit, geleitet. Der Ionenaustauscher wird abgetrennt und mit Salpetersäure regeneriert, wobei die Metallionen ausgewaschen (eluiert) werden und der Ionenaustauscher regeneriert wird. Das Eluat wird anschließend über einen mit Methylammoniumphosphongruppen modifizierten Ionenaustauscher (z.B. Polystyrolharz) geleitet. Dabei werden selektiv die Eisenionen nahezu quantitativ aus dem Eluat abgetrennt und die Kupfer- und Vanadiumionen verbleiben in Lösung.

Diese Lösung kann direkt wieder zur oxidativen Herstellung von Adipinsäure verwendet werden.

Das erfindungsgemäße Verfahren kann kontinuierlich oder diskontinuierlich durchgeführt werden.

Bei der Durchführung des erfindungsgemäßen Verfahrens wird zuerst mit einem Kationenaustauscher wie z.B. Lewatit SP 112 eine Abtrennung aller Metalle aus dem Reaktionsgemisch vorgenommen; dies geschieht bevorzugt dort im Adipinsäure-Aufarbeitungsprozeß, wo die Salpetersäure-Konzentration unter 10 % liegt. Das durch Regeneration des Ionenaustauschers mit Salpetersäure, bevorzugt 20 bis 30 %ig, gewonnene metällhaltige Regenerat wird anschließend bei den gegebenen sauren Bedingungen über einen Alkylaminophosphonsäuregruppen-haltigen Ionenaustauscher gegeben, wobei zuerst auch Kupfer und Vanadin aufgenommen werden, dann aber das Eisen trotz seiner um Zehnerpotenzen niedrigeren Konzentration Kupfer und Vanadin wieder verdrängt.

Beide Ionenaustausch-Schritte lassen sich zwischen 5°C und 100°C, bevorzugt zwischen 40°C und 75°C, durchführen.

Vorzugsweise schafft man an einem Behälter, der die Regeneriersäure enthält, einen Kreislauf, in dem die Eisenentfernung durch Umpumpen durch den Aminophosphonsäure-haltigen Ionenaustauscher erfolgt. Die enteisente Regeneriersäure kann mehrfach zur Regenerierung des ersten Ionenaustauschers benutzt werden. Da zum Nachspülen Frischsäure (20-30 %ige HNO₃) benutzt wird, läßt sich der pH zwischen 0 und -1,5 einfach einstellen.

### Beispiele

Die eingesetzten Regeneriersäuren enthaltenen Metalle z.B. in folgenden Konzentrationen:

| | | |
|---|---|---|
| Kupfer (Cu) | 5 - 35 g/l, | bevorzugt 10 - 28 g/l |
| Vanadin (V) | 0,5 - 10 g/l, | bevorzugt 1 - 6 g/l |
| Eisen (Fe) | 0,02-2,5 g/l, | bevorzugt 0,040 - 250 g/l |

Ist die Kapazität des Methylaminophosphonsäure-Ionenaustauschers erschöpft, dann kann die im Harzbett verbliebene Lösung mit einem Bettvolumen (Bv) Frischsäure (6 - 31,5 % n HNO₃, vorzugsweise 9 bis 16 % HNO₃), ausgespült werden, so daß ein Katalysatormetallverlust vollständig vermieden wird. Der Ionenaustauscher ist mit 5 - 50 %iger Phosphorsäure regenerierbar.

### Beispiel 1

Bei einer Beladungsgeschwindigkeit von ca. 10 BV/h werden 300 ml Lewatit SP112® mit ca. 6 BV einer Lösung, die 6 g/l Cu, 1,66 g/l V und 68 mg/l Fe enthielt, beladen (T=50°C). Das Eluat enthält <10 mg/l Cu, <10 mg/l V und <20 mg/l Fe.

Die Regenerierung des Ionenaustauschers wird mit 3 n HNO₃ durchgeführt. Einsatz 3 BV HNO₃ und 1 BV H₂O. Die Regeneriersäure enthält Cu = 11,21 g/l, V = 0,82 g/l, Fe = 42 mg/l.

Die Regeneriersäure wird 3 h durch eine 100 ml Methylaminophosphonsäuremodifiziertes Polystyrolharz (Ionenaustauscher) enthaltende Säule mit einer Geschwindigkeit von 600 ml/h im Kreis gefahren. Anschließend Spülung mit 100 ml 3 n HNO₃. Das Eluat enthält 8,4 g/l Cu, 62 mg/l V, <1 mg/l Fe.

### Beispiel 2

500 ml Regeneriersäure, hergestellt wie in Beispiel 1, werden mit 15 ml Methylaminophosphonsäure-Austauscherharz bei Raumtemperatur gerührt. Metall-Konzentrationen:
- Ausgangslösung:: 4,1 g/l V, 320 mg/l Fe, 16 g/l Cu
- Endkonzentration nach 10 h:: 4,0 g/l V, <23 mg/l Fe, 16 g/l Cu

### Beispiel 3

1 l Regeneriersäure, hergestellt wie in Beispiel 1, wird mit 40 ml Methylaminophosphonsäure-Harz bei Raumtemperatur 48 h gerührt.

| | Cu | V | Fe |
|---|---|---|---|
| Ausgangskonzentration: | 28 g/l | 15,0 g/l | 610 mg/l |
| Endkonzentration | 27 g/l | 13,0 g/l | 33 mg/l |

### Beispiel 4

Eine wie im Beispiel 1 hergestellte Regeneriersäure wird mit Cu (NO₃)₂, V₂O₅ und Fe(NO₃)₃·H₂O so angereichert, daß eine Ausgangsmetall-Konzentration von 28 g/l Cu, 15 g/l V und 610 mg/l Fe entsteht. Diese Lösung wird 48 h mit 20 ml Methylaminophosphonsäure-Harz gerührt. Endkonzentration der Lösung: 27 g/l Cu, 13 g/l V, 33 mg/l Fe.

### Beispiel 5

250 ml Regeneriersäure, hergestellt wie im Beipsiel 1, wird durch 74 ml Methylaminophosphonsäure-Harz gepumpt.
- Ausgangskonzentration:: 10 g/l Cu, 2,5 g/l V, 1,5 g/l Fe
- Endkonzentration nach ca. 36 h:: 10 g/l Cu, 2,2 g/l V, 55 mg/l Fe

### Beispiel 6

1 l Regeneriersäure (pH - 1,6) wurde mit 105 ml Methylaminophosphonsäure-Harz bei Raumtemperatur gerührt.
- Ausgangskonzentration:: 15 g/l Cu, 3,6 g/l V, 2,3 g/l Fe
- Endkonzentration nach ca. 24 h:: 15 g/l Cu, 3,6 g/l V, 180 mg/l Fe

### Beispiel 7

1,5 l Regeneriersäure, hergestellt wie im Beispiel 1, werden durch 45 g feuchtes Austauscherharz Lewatit VP OC 1060® gepumpt.

Metalle in der Ausgangslösung:
13 g/l Cu = 19,5 g Cu; 3,2 g/l V = 4,8 g V; 270 mg/lFe = 405 mg Fe

Man erhält 1,44 l Eluat mit den absoluten Metallgehalten:
Cu = 16,81 g; V = 3,99 g; Fe = 4,7 mg

Intensives Spülen mit 350 ml 1,5 n HNO₃ bringt Cu = 2,8 g; V = 0,825 g; Fe = 1,4 mg

## Patentansprüche

1. Verfahren zur Entfernung von Eisenionen aus einer Reaktionslösung wie sie bei der Oxidation von Cyclohexanol mit Salpetersäure zu Adipinsäure mit konzentrierter Salpetersäure in Gegenwart von Kupfer- und Vanadiumsalzen anfällt, dadurch gekennzeichnet, daß man die Reaktionslösung nach Abtrennen der Adipinsäure über einen sulfonierten Ionenaustauscher leitet, wobei alle Metallionen absorbiert werden, daß man anschließend den beladenen Ionenaustauscher abtrennt, mit Salpetersäure alle absorbierten Metallionen auswäscht und das Eluat auf einen mit Aminophosphonsäuregruppen modifizierten Ionenaustauscher aufgibt, wobei die Eisenionen selektiv abgetrennt werden.

## Claims

1. Process for the removal of iron ions from a reaction solution as obtained in the oxidation of cyclohexanol with nitric acid to yield adipic acid with concentrated nitric acid in the presence of copper and vanadium salts, characterised in that, once the adipic acid has been separated, the reaction solution is passed through a sulfonated ion exchanger, wherein all metal ions are absorbed, that the loaded ion exchanger is then separated, all the absorbed metal ions are eluted with nitric acid and the eluate applied onto an ion exchanger modified with aminophosphonic acid groups, wherein the iron ions are selectively separated.

## Revendications

1. Procédé de séparation des ions fer d'une solution de réaction, telle qu'elle se forme lors de l'oxydation du cyclohexanol en acide adipique avec de l'acide nitrique concentré, en présence de sels de cuivre et de vanadium, caractérisé en ce que l'on envoie la solution de réaction après séparation de l'acide adipique, sur une échangeuse d'ions sulfonée, où tous les ions métalliques sont absorbés, qu'ensuite, on sépare l'échangeuse d'ions chargée, on lave tous les ions métalliques absorbés au moyen d'acide nitrique et on charge l'éluat sur une échangeuse d'ions modifiée par des radicaux acide aminophosphonique, où les ions fer sont sélectivement séparés.
